(19) Europäisches Patentamt
European Patent Office
Office européen des brevets

(11) Publication number: 0 102 046
A1

(12) # EUROPEAN PATENT APPLICATION

(21) Application number: 83108240.9

(22) Date of filing: 22.08.83

(51) Int. Cl.[3]: C 07 D 221/10
C 07 D 221/16, C 07 D 491/04
C 07 D 495/04, C 07 D 471/04
C 07 D 491/12, C 07 D 495/12
A 61 K 31/435

(30) Priority: 26.08.82 US 411764
16.06.83 US 505050

(43) Date of publication of application: 07.03.84 Bulletin 84/10

(84) Designated Contracting States: AT BE CH DE FR GB IT LI NL SE

(71) Applicant: SCHERING CORPORATION
2000 Galloping Hill Road
Kenilworth, New Jersey 07033(US)

(72) Inventor: Smith, Elizabeth Melva
166 Grove Avenue
Verona New Jersey 07044(US)

(72) Inventor: Doll, Ronald James
126 Union Avenue
Maplewood New Jersey 07040(US)

(72) Inventor: Neustadt, Bernard Ray
24 Brook Place
West Orange New Jersey 07052(US)

(74) Representative: Antony, Fritz, Dr. et al,
P.O. Box 601 Winkelriedstrasse 35
CH-6002 Lucerne(CH)

(54) **Tricyclic lactams, method for making them, pharmaceutical compositions containing them.**

(57) Compounds of the following formula are disclosed:

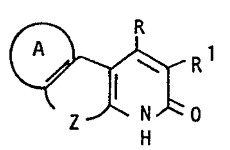

in which R is hydrogen or lower alkyl
$R_1$ is hydrogen, or one of various substituents
Z is $-CH=CH-$, $-(CH_2)_n-$ (n = 1,2,3, or 4) $-(CH_2)_p S(O)_m (CH_2)_r$ or $-(CH_2)_p O(CH_2)_r$

(m=0,1 or 2, p and r are independently 0,1,2 or 3 provided r + p = 0,1,2 or 3)
A is a substituted or unsubstituted benzo or pyrido ring, and the pharmaceutically acceptable salts thereof.
The compounds increase cardiac contractility.

EP 0 102 046 A1

TRICYCLIC LACTAMS,
METHOD FOR MAKING THEM,
PHARMACEUTICAL COMPOSITIONS CONTAINING THEM

This invention relates to certain tricyclic lactams which are useful in increasing cardiac contractility and to pharmaceutical compositions containing such compounds.

The compounds of the present invention are represented by the formula

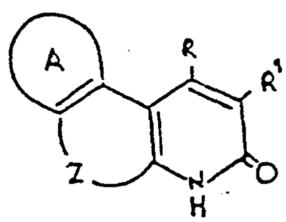

I

wherein R is hydrogen or lower alkyl;

$R^1$ is hydrogen, hydroxy, lower alkoxy, cyano, lower alkyl, halogen, $-NR^2R^3$, $-CONR^2R^3$, (wherein $R^2$ and $R^3$ are independently hydrogen, lower alkyl or hydroxy-lower-alkyl) $-NHCOR^2$, $-COOR^2$ (wherein $R^2$ is as defined above), or

$-\overset{M}{\overset{\|}{C}}NH_2$ wherein M is sulfur or NH; with the

proviso that R and $R^1$ are not both hydrogen;

Z is 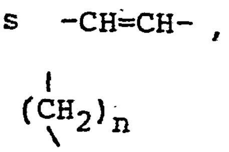

wherein n is 1,2,3 or 4,
$-(CH_2)_p-S(O)_m-(CH_2)_r$, or
$-(CH_2)_p-O-(CH_2)_r$

wherein m is 0,1 or 2, and p and r are independently 0,1,2 or 3 subject to the proviso that r + p is 0,1,2 or 3 ;

A represents a substituted or unsubstituted benzo or pyrido ring which when substituted have 1 to 3 substituents independently selected from halogen, hydroxy, lower alkyl and lower alkoxy;

and pharmaceutically acceptable salts thereof.

Unless otherwise stated, the term "lower alkyl" includes branched- and straight-chain alkyl groups of from 1 to 6 carbon atoms and includes, for example, methyl, ethyl, n-propyl, isopropyl, t-butyl and n-hexyl.

The term "lower alkoxy" includes both branched and straight-chain alkoxy groups of 1 to 6 carbon atoms and includes for example methoxy, ethoxy, isopropoxy, t-butoxy and n-hexoxy.

The term "halo" or "halogen" means chloro and bromo.

The term "pharmaceutically acceptable salts" includes the pharmaceutically acceptable acid addition salts of the compounds of formula I derived from a variety of organic and inorganic acids, such as sulfuric, phosphoric, hydrochloric, hydrobromic, sulfamic, citric, lactic, oleic, succinic, tartaric, cinnamic, acetic, benzoic, ascorbic, and other organic and inorganic acids which form pharmaceutically acceptable addition salts.

The nomenclature employed throughout this specification has been assigned from Parent Compound Handbook (American Chemical Society).

The term "A is a pyrido ring" means one of the following rings:

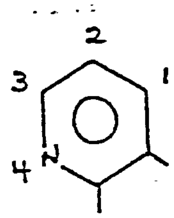

4-pyrido

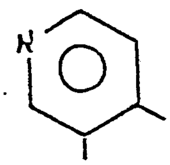

3-pyrido

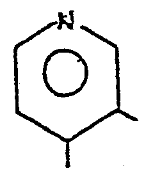

2-pyrido

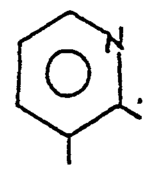

1-pyrido

each unsubstituted or substituted as mentioned above and with conventional numbering employed therewith.

The 2-oxo-pyridine derivatives (2(1H)-pyridone derivatives) are tautomeric with 2-hydroxy-pyridine derivatives as shown in the partial formulas below:

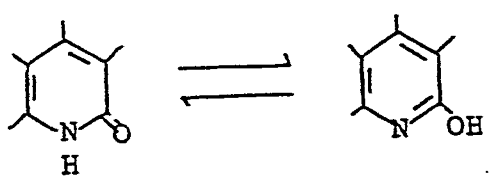

Throughout this specification, the "pyridone" term (e.g. pyridin-3-one) shall be used to mean any of the tautomers.

Preferred R lower alkyl groups are methyl, ethyl, n-propyl and isopropyl. Most preferably R is hydrogen or methyl.

Preferred $R^1$ groups are hydroxy, lower alkoxy, cyano, $-NR^2R^3$, $-CONR^2R^3$ and $-NHCOR^2$ wherein $R^2$ and $R^3$ are as defined above.

More preferred $R^1$ groups are cyano, amino and

$-\overset{\overset{O}{\|}}{C}NH_2$.

In some preferred compounds of the invention R is hydrogen while $R^1$ is cyano.

Preferably A is a substituted or unsubstituted benzo ring or a 3-pyrido ring.

Preferred lower alkyl groups for substituting the benzo or pyrido rings are methyl, ethyl, isopropyl and _n_-propyl.

Preferred lower alkoxy groups for substituting the benzo or pyrido rings are methoxy, ethoxy, isopropoxy and n-propoxy.

It is particularly preferred for the pyrido or benzo ring to be substituted with 1 to 3 substituents independently selected from chloro, bromo, amino, methoxy, methyl and hydroxy.

Of the above preferred compounds, those wherein Z is methylene, $-CH_2-CH_2-$,$-CH=CH-$,$-OCH_2-$,$-SCH_2-$, and $-SO_2CH_2-$ are particularly preferred, and most preferably Z is either $-CH_2-CH_2-$ or $-CH=CH-$.

Preferred compounds of this invention include 2- cyano -5,6-dihydro-benzo [f] quinolin-3(4H)-one and 2-cyanobenzo[f] quinolin-3(4H)-one.

The compounds of this invention increase cardiac contractility and are useful in the treatment of congestive heart failure.

*In vitro* and *in vivo* tests have been conducted with compounds of this invention demonstrating their ability to increase cardiac contractility.

*In vitro* tests were conducted on left atria obtained from guinea pigs employing the method described in J. Pharmacol. Exp. Ther. 217, 708-713 (1981). Increases in cardiac contractility were found at concentrations ranging from 1 μgm/ml to 32 μgm/ml.

*In vivo* tests were conducted on open-chest barbiturated-anaesthetized dogs employing the method described in J. Pharmacology Exp. Ther. 218, 444-452 (1981). Increases in cardiac contractility were generally found at dosage levels of 0.1 mg/kg to 3.2 mg/kg.

The compounds are administerable in a variety of formulations. Preferably the compounds will be administered parenterally. The compounds may be combined with any suitable pharmaceutical carrier and administered in a variety of formulations. The compounds may also be administered transdermally.

Typically, a daily dosage regimen would generally be 0.1 to 10 gm/day parenterally. Preferably, the compounds of this invention should be administered as a constant infusion titrated to the desired clinical effect. The actual dose to be administered is determined by the clinician and is dependent upon various factors such as the particular compound employed, age and weight of the subject, the severity of the disease and the individual's response. The daily dosage

may be administered either singly or divided proportionally into several dosages.

The compounds of formula I are generally made by a process characterized by reacting a compound of formula II

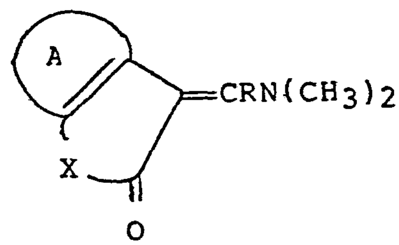

II

in which R and A are as defined above, and X is $-(CH_2)_n-$ or $-(CH_2)_p-O-(CH_2)_r$ or $(CH_2)_p-S-(CH_2)_r$ wherein n, p and r are as defined above, with a compound of formula

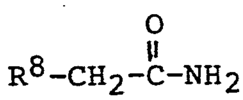

III

in which $R^8$ is hydrogen, cyano, loweralkoxy or loweralkyl under basic conditions to form a compound of formula $I_a$

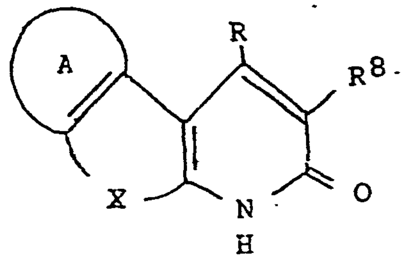

I a

(and when R and $R^8$ are both hydrogen, halogenating the compound of formula $I_a$ to produce a compound of formula I in which $R^1$ is halo-

gen) whereby a compound of formula I is obtained in which A,R, and X are as defined above and $R^1$ is halogen, hydrogen, cyano, loweralkoxy or loweralkyl, and if desired, thereafter converting such compound of formula I into another compound of formula I by a known method, and isolating the compound of formula I as such or as a pharmaceutically acceptable salt.

The process of the invention described herein thus involves a cyclization reaction between compound II and a monosubstituted acetamide III.

This cyclization reaction may be accomplished by addition of 1 to 1.5 equivalents of the substituted acetamide III to II. The reaction is usually conducted in the liquid phase employing an inert organic solvent such as dimethylformamide. An excess (3 equivalents) of a base such as sodium methoxide, sodium ethoxide and the like may be employed in the system. Reaction pressure is not critical and for convenience the reaction is generally conducted at from 60° C to 100° C and is generally complete from within 2 to 10 hours.

The product may then be isolated and purified by conventional procedures such as extraction, filtration, chromatography, distillation, or alternatively, may be used in succeeding reactions without isolation and/or purification.

..... / .....

The conversion of a compound of formula I into another compound of formula I as mentioned above can be accomplished by various procedures recognized in the art, but will generally involve one or more of the following facultative steps:

a) oxidation of an $-CH_2CH_2-$ group Z to $-CH=CH-$;

b) oxidation of a group $-(CH_2)_p-S-(CH_2)_r-$ to $-(CH_2)_p-S(O)_{m'}-(CH_2)_r$ wherein p and r are as defined above and m' is 1 or 2

c) conversion of a cyano group $R^1$ to carboxamide;

d) conversion of a carboxamide group $R^1$ to amino;

e) acylation of an amino group $R^1$;

f) hydrolysis of a cyano group $R^1$ to carboxy;

g) esterification of a carboxy group $R^1$;

h) conversion of an ester group $R^1$ into a carboxamido group $-\overset{O}{\overset{\|}{C}}NR^2R^3$ in which $R^2$ and $R^3$ are as defined above;

i) decarboxylation of a compound of formula I in which $R^1$ is carboxyl;

j) halogenation of a compound of formula I in which $R^1$ is hydrogen;

k) hydrolysis of a compound of formula I in which $R^1$ is halide;

l) amidation or amination of a compound of formula I in which $R^1$ is halogen;

m) converting a cyano group $R^1$ to the thiocarboxamide group;

n) converting a cyano group $R^1$ to a carboxamidine group.

In particular the 2-cyanopyridin-3-(4H)-ones, A, are readily derivatized by procedures recognized in the art

for example as shown in the following flow diagram, wherein $X'_2$ is either $Cl_2$ or $Br_2$.

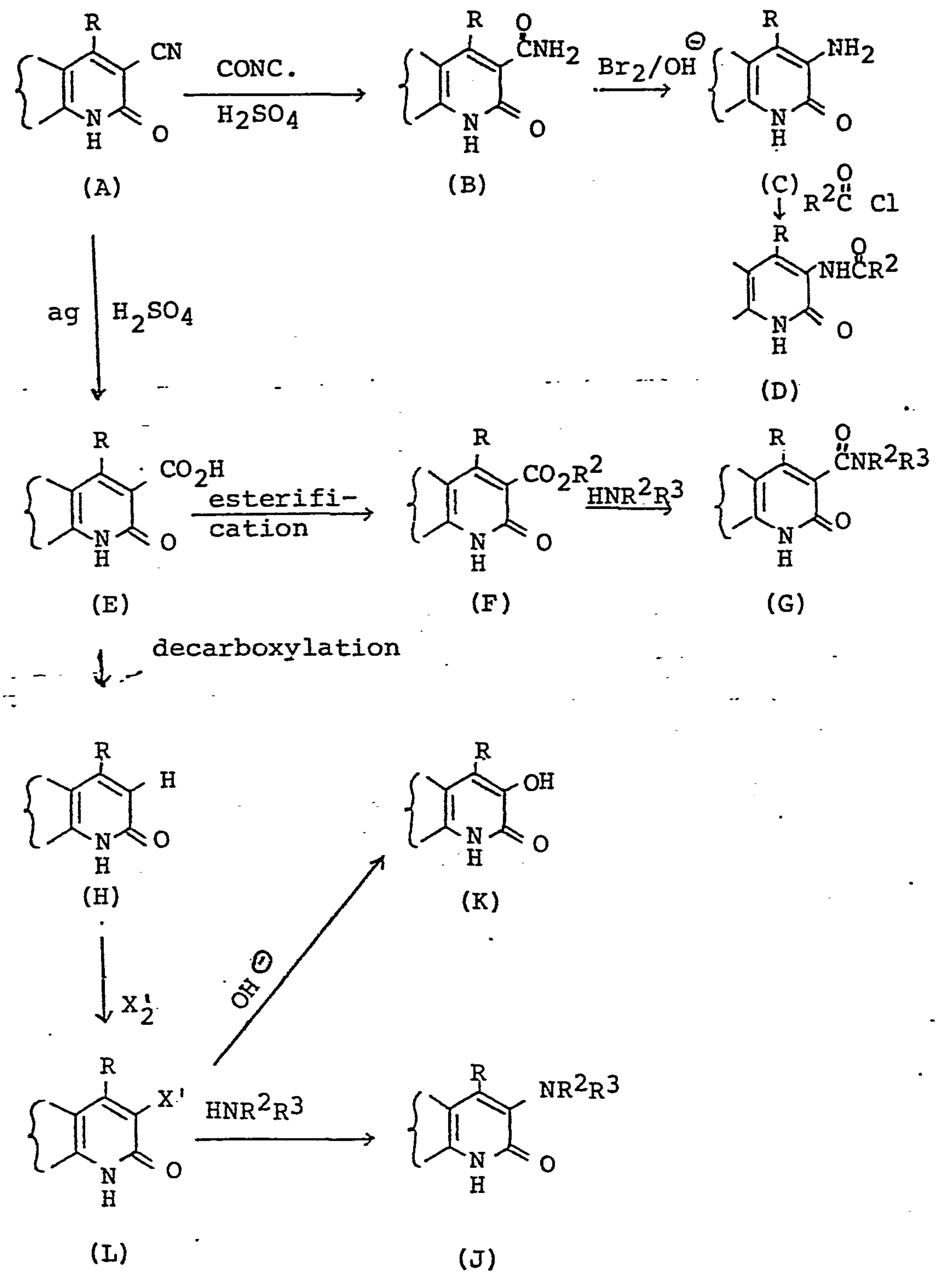

The 3-cyanopyridin-2(1H)-one, A, is readily converted to the carboxamide, B, by treatment with concentrated acid such as concentrated sulfuric acid. The carboxamide may then be converted to the amine, C, by treatment with bromine and base. The amine may then be acylated by reaction with an acid halide to form D.

The 3-cyanopyridin-2(1H)-one A may also be hydrolyzed to the carboxylic acid, B, by treatment with aqueous acid such as aqueous sulfuric acid. The carboxylic acid may then be readily esterified under acidic or basic conditions to form the ester, F.

The ester F may be converted to the carboxamide, G, by treatment with an excess of ammonia or the appropriate amine in an inert hydroxylic solvent, water, or a mixture of water and an inert hydroxylic solvent.

The 3-carboxylic acid E may be readily decarboxylated by heating to produce H which may then be treated with chlorine or bromine to produce the 3-halopyridin-2(1H)-one, L.

The 3-halopyridin-2(1H)-one, L, may be treated with ammonia or a mono or diloweralkylamine to produce the 3-amino or 3-mono or diloweralkylamino product.

A suitable method for the conversion of the 3-halogen to the 3-hydroxy-pyridin-2(LH)-one, K, is described in U.S. Patent 4225601, column 3, that is by reacting the 3-halide with an alkali metal lower alkoxide in a lower alkanol, e.g. sodium methoxide in methanol, autoclaving at about 200$^{o}$ C.

The 3-cyanopyridin-2(1H)-ones, VII, may be converted to the thiocarboxamide group as shown in reaction (A) below:

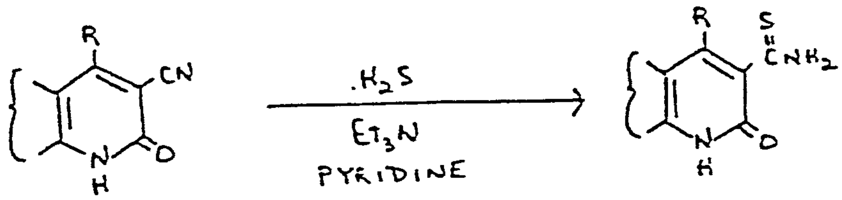


(A)

VII VIII

wherein R is as defined above.

The reaction is known in the art and is described by J. Chem. Soc., 742-744 (1952). The reaction is accomplished by treating the 3-cyanopyridin-2(1H)-one, VII, with hydrogen sulfide and triethylamine in pyridine at or above room temperature. The product, VIII, is then isolated and purified by conventional procedures such as extraction, distillation, chromatography, filtration and the like.

The 3-cyanopyridin-2(1H)-ones, VII, may also be converted to the carboxamidine group by treatment with liquid ammonia and ammonium chloride at elevated temperatures (e.g.80° C) as shown in reaction (B) below:

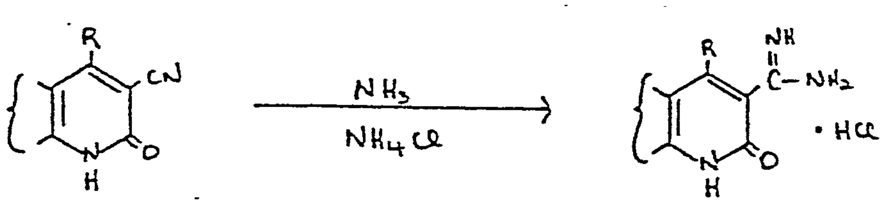


(B)

VII IX

A 'Z'group $-CH_2-CH_2$ can be oxidised to -CH=CH- by any suitable method known in the art, for example by reacting a compound of formula I in which Z is $-CH_2CH_2-$, preferably in an organic solvent e.g. dioxane, with an oxidising agent for example 2,3-dichloro-5,6-dicyano-1,4-benzoquinone, at elevated temperature e.g. at the reflux temperature of the solvent.

A 'Z'group $-(CH_2)_p-S-(CH_2)_r$ can be oxidized by any suitable S-oxidation method known in the art, for example by reacting a compound of formula I in which Z is $-(CH_2)_p-S-(CH_2)_r$ usually in an organic solvent e.g. chloroform with a mild oxidizing agent for example m-chloro-benzoic acid, usually at room temperature.

The compounds of formula II are generally prepared as shown in the following reaction

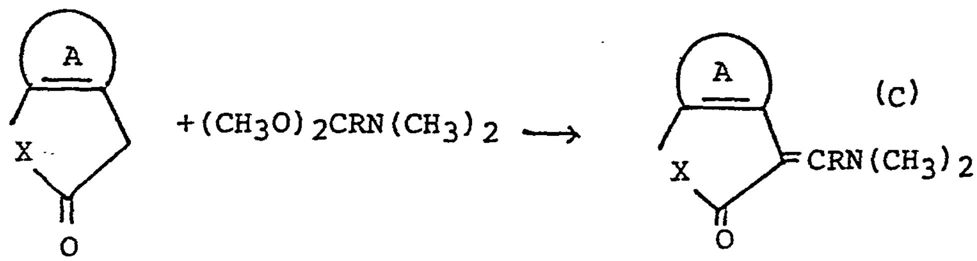


IV V II

in which A, R and X are as defined above.

This reaction comprises the condensation of an amide acetal V with the active ketone IV, to give II. For example, the reaction can be conducted by adding an essentially equimolar amount of N,N-dimethylformamide dimethyl acetal, to IV . The reaction is generally conducted neat (i.e., without solvent). Reaction pressure is not critical and for convenience the reaction is generally conducted at atmospheric pressure. The reaction is generally conducted at reflux and is generally complete from within 1/2 to 4 hours. The product II may then be isolated and purified by conventional procedures such as extraction, filtration, chromatography, distillation, or alternatively, may be used in succeeding reactions without purification.

Various starting materials for the reaction (c) e.g. 2-tetralone, substituted 2-tetralones, 2-indanone, benzocycloheptan-2-one, benzocyclooctan-2-one and N,N-dimethylformamide dimethyl acetal, are known in the art.

Other starting materials can be prepared by methods known in the art, for instance 5,6,7,8-tetrahydro-quinolin-7 (or 6)-one; 5,7-dihydrocyclopenta[c]pyridin-6-one; and 5,6,7,8,9,10-hexahydrocylcooctan[1,2-b] pyridin-3(4H)-one and starting materials of similar structure can be prepared according to the general procedure reported by Maeda and Ohsugi, Chem. Pharm. Bull., 16, 897-908 (1968).

The following examples describe in detail the preparation of the compounds and compositions of the present invention. As used herein, the term "room temperature" refers to about 20° C to 25° C. Unless otherwise stated, all temperaturesand temperature ranges are in degrees Celsius.

PREPARATION 1 1-Dimethylaminomethylene-2-tetralone

Heat 8.0 ml of 2-tetralone and 12.0 ml of dimethylformamide dimethyl acetal under reflux at 80° for 1 1/2 hours. Concentrate the reaction mixture *in vacuo*. Dissolve the residue in hot methanol, add charcoal, filter, and concentrate the filtrate *in vacuo* to give the title compound, an amber oil.

PREPARATION 2 1-Dimethylaminomethylene-1,4-dihydro-3-benzopyran-2-one

Heat 8.8 gm of 1,4-dihydro-3-benzopyran-2-one and 12.0 ml of dimethylformamide dimethyl acetal under reflux for 1 1/2 hours. Concentrate the reaction mixture *in vacuo*. Dissolve the residue in hot methanol, add charcoal, filter, and concentrate the filtrate *in vacuo* to give the title compound.

EXAMPLE 1 2-Cyano-5,6-dihydro-benzo[f]-quinolin-3(4H)-one

Heat 11.4 g of 1-dimethylaminomethylene-2-tetralone, 4.46 gm of cyanoacetamide and 6.6 gm of sodium methoxide in 100 ml of dimethylformamide for 8 hours at 80°. Cool and dilute the reaction mixture with 150 ml of acetonitrile. Remove the tan solid by filtration. Dissolve the solid in hot water and filter the mixture. Acidify the aqueous solution with concentrated hydrochloric acid to give a tan precipitate. Filter and wash the tan solid with chloroform to give the title compound, m.p. 301-3°. Concentrate the remaining dimethylformamide:acetonitrile solution *in vacuo* to give a green-black residue and chromatograph on 800 ml of silica gel using chloroform:methanol 98:2 as eluant to give an additional amount of the title compound (total yield 19%).

EXAMPLE 2 2-Cyano-benzo[f]quinolin-3(4H)-one

Add 11.1 gm of 2-cyano-5,6-dihydrobenzo[f]-

quinolin-3(4H)-one to 1500 ml of anhydrous 1,4-dioxane containing 33.0 gm of DDQ (2,3-dichloro-5,6-dicyano-1,4-benzoquinone). Heat the system at reflux for 16 hours. Filter the solution and isolate the title compound crystallizing from methanol as a yellow crystalline solid m.p. 300° C. (yield 30%).

EXAMPLE 3 2-Cyano-4,6-dihydro-3H-[2]Benzopyrano[3,4-b]pyridin-3-one

Heat 5.7 gm of 1-dimethylaminomethylene 1,4-dihydro-3-benzopyran-2-one, 2.23 gm of cyanoacetamide and 3.3 gm of sodium methoxide in 100 ml of dimethylformamide for 8 hours at 80°. Cool and dilute the reaction mixture with 150 ml of acetonitrile and filter the mixture to isolate the title compound.

Similarly the following compounds of this invention may be prepared by using the procedure of the above examples and employing the appropriate reagents:

3-cyano-1,9-dihydro-2H-cyclopenta[1,2-b:4,3-b']-dipyridin-2-one;

3-ethyl-1,9-dihydro-2H-cyclopenta[1,2-b:4,3-b']-dipyridin-2-one;

3-cyano-1,9-dihydro-2H-indeno[2,1-b]pyridin-2-one;

1,9-dihydro-4-methyl-2H-cyclopenta[1,2-b:4,3-c']-dipyridin-2-one;

3-cyano-1,9-dihydro-2H-cyclopenta[1,2-b:4,3-c']-dipyridin-2-one;

3-cyano-4-methyl-1,9-dihydro-2H-cyclopenta-[1,2-b:4,3-c']dipyridin-2-one;

3-ethoxy-1,9-dihydro-5,6,8-trimethyl-2H-cyclopenta [1,2-b:3,4-c']dipyridin-2-one;

3-cyano-1,9-dihydro-2H-cyclopenta[1,2-b:3,4-c']-dipyridin-2-one;

3-cyano-4-ethyl-1,9-dihydro-2H-cyclopenta-[1,2-b:3,4-b'] dipyridin-2-one;

3-cyano-1,9-dihydro-2H-cyclopenta[1,2-b:3,4-b']-dipyridin-2-one;

2-cyanobenzo[f]quinolin-3(4H)-one;

1-n-propylbenzo[f]quinolin-3(4H)-one;

2-cyano-5,6-dihydrobenzo[f]quinolin-3(4H)-one;

5,6,-dihydro-2,8-dimethoxybenzo[f]-quinolin-3(4H)-one;

2-cyano-5,6,-dihydro-1-methylbenzo[f]quinolin-3(4H)-one;

9-cyano-1,7-phenanthrolin-8(7H)-one;

9-cyano-4,7-phenanthrolin-8(7H)-one;

9-methoxy-2,3-dimethyl-4,7-phenanthrolin-8(7H)-one;

9-cyano-3,7-phenanthrolin-8(7H)-one;

9-cyano-1,4-dimethyl-2,7-phenanthrolin-8(7H)-one;

9-cyano-5,6-dihydro-4,7-phenanthrolin-8(7H)-one;

9-cyano-5,6-dihydro-1,7-phenanthrolin-8(7H)-one;

9-cyano-5,6,-dihydro-3,7-phenanthrolin-8(7H)-one;

9-cyano-5,6-dihydro-2,7-phenanthrolin-8(7H)-one;

5,6-dihydro-9-methoxy-1,3-dimethyl-2,7-phenanthrolin-8(7H)-one;

5,6-dihydro-10-methyl-2,7-phenanthrolin-8(7H)-one;

2-cyano-4,5,6,7-tetrahydro-3H-benzo[3,4]cyclohepta[1,2-b]pyridin-3-one;

10-cyano-5,6,7,8-tetrahydro-9H-cyclohepta-[1,2-b:4,3-b']dipyridin-9-one;

5,6,7,8-tetrahydro-10-methoxy-1,2,3-trimethyl-9H-cyclohepta-[1,2-b:4,3-b']dipyridin-9-one;

10-cyano-5,6,7,8-tetrahydro-9H-cyclohepta-[1,2,-b:4,3-c']dipyridin-9-one;

5,6,7,8-tetrahydro-10-methoxy-1,2,4-trimethyl-9H-cyclohepta-[1,2-b:4,3-c']dipyridin-9-one;

5,6,7,8-tetrahydro-10-methoxy-1,2,3-trimethyl-9H-cyclohepta-[1,2-b:3,4-b']dipyridin-9-one;

10-cyano-5,6,7,8-tetrahydro-9H-cyclohepta-[1,2-b:3,4-c']dipyridin-9-one;

10-cyano-5,6,7,8-tetrahydro-9H-cyclohepta-[1,2-b:3,4-b']dipyridin-9-one;

3-cyanofuro[2,3-b:5,4-c']dipyridin-2(1H)-one;
3-cyanofuro[2,3-b:5,4-b']dipyridin-2(1H)-one;
3-cyano-4-methylfuro[2,3-b:5,4-c']dipyridin-2(1H)-one;
3-cyanofuro[2,3-b:4,5-c']dipyridin-2(1H)-one;
3-cyanofuro[2,3-b:4,5-b']dipyridin-2(1H)-one;
2-cyano-4,6-dihydro-3H-[2]benzopyrano[3,4-b]-pyridin-3-one;
2-cyano-4,6-dihydro-1-n-propyl-3H-[2]benzopyrano-[3,4-b] pyridin-3-one;
2,8-diethoxy-4,6-dihydro-3H-[2]benzopyrano-[3,4-b]pyridin-3-one;
2-cyano-4,6-dihydro-1-methyl-3H-pyrano[2,3-b:5,4-b']-dipyridin-3-one;
2-cyano-4,6-dihydro-3H-thiopyrano[2,3-b:5,4-b']-dipyridin-3-one;
9-cyano-5,7-dihydro-3H-thiopyrano[2,3-b:5,4-b']-dipyridin-8-one;
9-cyano-5,7-dihydro-8H-thiopyrano[2,3-b:4,5-c']-dipyridin-8-one;
9-cyano-5,7-dihydro-8H-thiopyrano[2,3-b:4,5-b']-dipyridin-8-one;
5,7-dihydro-2,3-dimethyl-9-ethoxy-8H-pyrano-[2,3-b:4,5-b']dipyridin-8-one;
2-cyano-1-ethyl-5H-[1]benzothiopyrano[3,4-b]-pyridin-3(4H)-one;
2-cyano-5H-[1]benzopyrano[3,4-b]pyridin-3(4H)-one;
2-methoxy-6H-thiopyrano[2,3-b:5,4-b']-dipyridin-3-(4H)-one;
9-cyano-6H-pyrano[3,2-b:5,4-b']dipyridin-8(7H)-one;
2-cyano-6,7-dihydro-[3]benzothiepino[2,1-b]pyridin-3(4H)-one;
9-cyano-5,8-dihydro-10-methylthiepino[2,3-b:4,5-b']-dipyridin-9(6H)-one.

2-cyano-5,6,7,8-tetrahydrobenzo[3,4]cycloocta-[1,2-b]pyridin-3(4H)-one;

11-cyano-5,6,7,8-tetrahydrocycloocta-[1,2-b:4,3-c']-dipyridin-10(9H)-one;

3-cyano-[1]benzothieno[2,3-b]pyridin-2(1H)-one;

3-ethylthieno[2,3-b:5,4-b']dipyridin-2(1H)-one;

3-cyanothieno[2,3-b:5,4-b']dipyridin-2(1H)-one;

4-methylthieno[2,3-b:5,4-c']dipyridin-2(1H)-one;

3-cyanothieno[2,3-b:5,4-c']dipyridin-2(1H)-one;

3-cyano-4-methylthieno[2,3-b:5,4-c']dipyridin-2(1H)-one;

3-ethoxythieno[2,3-b:4,5-c']dipyridin-2(1H)-one;

3-cyanothieno[2,3-b:4,5-c']dipyridin-2(1H)-one;

3-cyano-4-ethylthieno[2,3-b:4,5-b']dipyridin-2(1H)-one;

3-cyanothieno[2,3-b:4,5-b']dipyridin-2(1H)-one;

3-cyanobenzofuro[2,3-b]pyridin-2(1H)-one;

2-cyano-6,7-dihydro-[3]benzoxepino[2,1-b]pyridin-3(4H)-one;

2-cyano-6,7-dihydro-oxepino[2,3-b:5,4-b']dipyridin-3(4H)-one;

2-cyano-5,7-dihydro-[2]benzothiepino[4,5-b]-pyridin-3(4H)-one;

10-cyano-5,7-dihydro-11-methylthiepino[3,4-b:6,5-c']-dipyridin-9(8H)-one;

2-cyano-5,7-dihydro-[2]benzoepino[4,5-b]pyridin-3(4H)-one:

10-cyano-5,7-dihydro-11-methyloxepino(3,4-b:5,6-c']-dipyridin-9(8H)-one;

2-cyano-5,6-dihydro-[1]benzothiepino[4,5-b]pyridin-3(4H)-one;

10-cyano-6,7-dihydro-11-methylthiepino[3,2-c:5,4-b']-dipyridin-9(8H)-one;

2-cyano-5,6-dihydro[1]benzoxepino[4,5-b]pyridin-3(4H)-one; and

2-cyano-5,6-dihydro-1-ethyloxepino[2,3-b:5,4-b']-dipyridin-3(4H)-one.

EXAMPLE 4 5,6-Dihydro-benzo[f]quinolin-3(4H)-one-2-carboxamide

Heat 5.5 gm 2-cyano-5,6-dihydrobenzo[f]-quinolin-3(4H)one in 50 ml of 90% sulfuric acid at 100° for 1 1/2 hours. Cool the reaction mixture, pour into ice water and basify the aqueous mixture with 50% sodium hydroxide to give a tan precipitate. Filter the mixture and recrystallize the solid from dimethylformamide: acetonitrile (volumetric ratio 1:1) to give the title compound m.p. 335-40° C.

EXAMPLE 5 5,6-Dihydro-benzo[f]quinolin-3(4H)-one-2-carboxamidine Hydrochloride

Add 2.10 gm of 2-cyano-5,6-dihydrobenzo[f]-

quinolin-3(4H)-one to 0.53 gm of ammonium chloride in liquid ammonia in a steel bomb. Heat the system to 80°C for 24 hours. Cool the system to room temperature and remove the ammonia to isolate 5,6-dihydrobenzo[f]quinolin-3(4H)-one-2-carboxamidine hydrochloride.

EXAMPLE 6 5,6-Dihydro-benzo[f]quinolin-3(4H)-one-2-thiocarboxamide

Add 4.20 gm of 2-cyano-5,6-dihydrobenzo[f]-quinolin-3(4H)-one to 50 gm of pyridine. Add 3.0 ml of triethylamine to the system. Cool the system to 0°-5°C and treat the system with hydrogen sulfide gas until the reaction is complete as indicated by thin layer chromatography. Remove the pyridine and triethylamine by stripping to isolate 5,6-dihydrobenzo[f]quinolin-3(4H)-one-2-thiocarboxamide.

EXAMPLE 7 5,6-Dihydro-benzo[f]quinolin-3(4H)-one-2-carboxylic acid

Add 6.3 gm of 2-cyano-5,6-dihydro-benzo[f]-quinolin-3(4H)-one to 100 ml of 50% aqueous sulfuric acid. Heat the system to reflux for 6 hours. Cool the reaction mixture, pour into ice water and isolate the title compound.

EXAMPLE 8 Methyl 5,6-Dihydro-benzo[f]quinolin-3(4H)-one-2-carboxylate

Add 6.0 gm of 5,6-dihydro-benzo[f]quinolin-3(4H)-one-2-carboxylic acid to 100 ml of methanol. Dropwise at $0^{O}C$, add 10 ml of thionyl chloride to the system. Heat the system at reflux for 6 hours. Remove the solvent by stripping to isolate the title compound (m.p. 290 - $3^{O}$ C).

EXAMPLE 9 5,6-Dihydro-benzo[f]quinolin-3(4H)-one-2-N,N-dimethylcarboxamide

Add 2.7 gm of methyl 5,6-dihydro-benzo[f]-quinolin-3(4H)-one-2-carboxylate to a steel bomb containing about 50 ml 40% aqueous dimethylamine. Heat the system in an oil bath at $100^{O}$ for 16 hours. Stop the reaction and cool the system to room temperature. Extract the product with chloroform and wash the chloroform solution with water. Treat the chloroform solution with charcoal and then filter. Dry the chloroform solution with anhydrous sodium sulfate and filter. Remove the solvent by stripping to give the title compound.

Similarly, the following compounds of this invention may be prepared by using the above procedures and employing the appropriate starting materials:

benzo[f]quinolin-3(4H)-one-2-carboxylic acid;

1,9-dihydro-2H-cyclopenta[1,2-b:4,3-b']-dipyridin-2-one-3-carboxylic acid;

methyl benzo[f]quinolin-3(4H)-one-2-carboxylate

1,9-dihydro-2H-cyclopenta[1,2-b:4,3-b']-dipyridin-2-one-3-carboxamide;

1,9-dihydro-2H-indeno[2,1-b]pyridin -2-one-3-thiocarboxamide;

1,9-dihydro-2H-indeno[2,1-b]pyridin -2-one-3-carboxamide;

1,9-dihydro-2H-cyclopenta[1,2-b:4,3-c']-dipyridin-2-one-3-carboxamide;

1,9-dihydro-2H-cyclopenta[1,2-b:3,4-c']-dipyridin-2-one-3-carboxamidine;

1,9-dihydro-2H-cyclopenta[1,2-b:3,4-b']-dipyridin-2-one-3-carboxamide;

benzo[f]quinolin-3(4H)-one-2-carboxamide;

benzo[f]quinolin-3(4H)-one-2-thiocarboxamide;

benzo[f]quinolin-3(4H)-one-2-carboxamidine;

5,6-dihydrobenzo[f]quinolin-3(4H)-one-2-carboxamide;

5,6-dihydrobenzo[f]quinolin-3(4H)-one-2-thiocarboxamidine;

5,6-dihydrobenzo[f]quinolin-3(4H)-one-2-carboxamidine;

1,7-phenanthrolin-8(7H)-one-9-carboxamide;

4,7-phenanthrolin-8(7H)-one-9-thiocarboxamide;

3,7-phenanthrolin-8(7H)-one-9-carboxamide;

5,6-dihydro-4,7-phenanthrolin-8(7H)-one-9-carboxamide;

5,6-dihydro-1,7-phenanthrolin-8(7H)-one-9-carboxamidine;

5,6-dihydro-10-methyl-3,7-phenanthrolin-8(7H)-one-9-carboxamide;

5,6-dihydro-3,7-phenanthrolin-8(7H)one-9-carboxamidine;

5,6-dihydro-2,7-phenanthrolin-8(7H)-one-9-(N,N-dimethyl)carboxamide;

4,5,6,7-tetrahydro-3H-benzo[3,4]cyclohepta[1,2-b]pyridin-3-one-2-thiocarboxamide;

5,6,7,8-tetrahydro-9H-cyclohepta[1,2-b:4,3-b']-dipyridin-9-one-10-(N,N-diethyl)carboxamide;

5,6,7,8-tetrahydro-9H-cyclohepta[1,2-b:4,3-c']-dipyridin-9-one-10-carboxamide;

5,6,7,8-tetrahydro-9H-cyclohepta[1,2-b:4,3-c']-dipyridin-9-one-10-carboxamidine;

5,6,7,8-tetrahydro-9H cyclohepta[1,2-b:3,4-b']- dipyridin-9-one-10-carboxamidine;

5,6,7,8-tetrahydro-9H-cyclohepta[1,2-b:3,4-c']dipyridin-9-one-10-thiocarboxamide;

[1]benzothieno[2,3-b]pyridin-2(1H)-one-3-carboxylic acid;

Methyl thieno[2,3-b:5,4-c']dipyridin-2(1H)-one-3-carboxylate;

benzofuro[2,3-b]pyridin-2(1H)-one-3-carboxamide;

furo [2,3-b:5,4-b']dipyridin-2(1H)-one-3-thiocarboxamide;

4,6-dihydro-3H-[2]benzothiopyrano[3,4-b]pyridin-3-one-2-carboxamide;

5,7-dihydro-8H-thiopyrano[2,3-b:4,5-c']dipyridin-8-one-9-carboxylic acid;

5,7-dihydro-8H-pyrano[2,3-b:4,5-b']dipyridin-8-one-9-carboxylic acid;

4H-[1]benzothiopyrano[3,4-b]pyridin-3(5H)-one-2-thiocarboxamide;

6H-thiopyrano[2,3-c:5,4-b']dipyridin-8(7H)-one-9-carboxamidine;

1-methyl-4H-[1]benzopyrano[3,4-b]pyridin-3(4H)-2-thiocarboxamide;

6H-pyrano[2,3-b:5,4-b']dipyridin-8(7H)-one-9-carboxamidine;

6,7-dihydro-1-ethyl-[3]benzothiepino[2,1-b]-pyridin-3(4H)-one-2-carboxylic acid;

6,7-dihydrothiepino[2,3-b:5,4-b']dipyridin-3(4H)-one-2-carboxamide;

4,6-dihydro-3H-[2]benzopyrano-[3,4-b]pyridin-3-one carboxamide hydrochloride;

4,6-dihydro-3H-[2]benzopyrano-[3,4-b] pyridin-3-one -2-carboxamidine hydrochloride;

4,6-dihydro-3H-[2]benzopyrano[3,4-b]pyridin-3-one -2-thiocarboxamide;

4,6-dihydro-3H[2]benzopyrano-[3,4-b] pyridin-3-one -2-carboxylic acid;

methyl 4,6-dihydro-3H[2]-benzopyrano [3,4-b]pyridin-3-one-2-carboxylate; and

4,6-dihydro-3H-[2]benzopyrano-[3,4-b]pyridin-3-one -2-N,N-dimethylcarboxamide;

Methyl 6,7-dihydro-[3]benzoxepino[2,1-b]-pyridin-3(4H)-one-2-carboxylate;

5,8-dihydro-oxepino[2,3-b:4,5-c']dipyridin-9(6H)-one-10-thiocarboxamide;

5,7-dihydro-[2]benzothiepino[4,5-b]pyridin-3(4H)-one-2-carboxamidine;

5,7-dihydrothiepino[3,4-b:5,6-b']dipyridin-9(8H)-one-10-carboxamide;

5,7-dihydro-[2]benzoxepino[4,5-b]pyridin-3(4H)-one-2-carboxylic acid;

6,7-dihydrothiepino[2,3-c:5,4-b']dipyridin-9(8H)-one-2-thiocarboxamide;

5,6-dihydro-oxepino[2,3-b:5,4-b']dipyridin-3(4H)-one-2-carboxamidine;

EXAMPLE 10 2-Amino-5,6-dihydro-benzo[f]-quinolin-3(4H)-one

Add 3.65g of 2-carbamoyl-5,6-dihydro-benzo[f]quinolin-3(4H)-one, to a solution of 4,2g of sodium hydroxide in 65 ml of water containing 1.05 ml of bromine at 0-5$^{o}$ C. Stir the resulting mixture at 100$^{o}$ for 3 hours. Cool and acidify the reaction mixture with concentrated hydrochloric acid. Stir this solution for 45 minutes and then cool this solution to give a precipitate. Filter the mixture and recrystallize the solid from methanol to give the title compound.

EXAMPLE 11 2-(N-acetylamino)-5,6-dihydro-benzo[f]-quinolin-3(4H)-one

To 2.0 gm of 2-amino-5,6-dihydro-benzo[f]quinolin-3(4H)-one, in 100 ml of chloroform containing 1 ml of pyridine add 0.80 gm of acetyl chloride. Stir the reaction mixture at room temperature for 5 hours. Filter and wash the solution with water. Remove the solvent by stripping to give the title compound.

Similarly, the following compounds of this invention may be prepared by using the above procedures and employing the appropriate starting materials:

3-amino-6-chloro-1,9-dihydro-2H-cyclopenta[1,2-b:4,3-b']dipyridin-2-one;

3-amino-1,9-dihydro-2H-indeno-[2,1-b]pyridin-2-one;

3-amino-1,9-dihydro-2H-cyclopenta[1,2-b:4,3-c']-dipyridin-2-one;

3-(N-acetylamino)-1,9-dihydro-2H-cyclopenta-[1,2-b:3,4-c']dipyridin-2-one;

3-amino-1,9-dihydro-2H-cyclopenta[1,2-b:3,4-b']-dipyridin-2-one;

2-aminobenzo[f]quinolin-3(4H)-one;

2-amino-8-chlorobenzo[f]quinolin-3(4H)-one;

2-(N-acetyl-N-methylamino)-5,6-dihydrobenzo[f]-quinolin-3(4H)-one;

2-(N-acetylamino)benzo[f]quinolin-3(4H)-one;

2-amino-8-chloro-5,6-dihydrobenzo[f]-quinolin-3(4H)-one;

9-amino-2-chloro-1,7-phenanthrolin-8(7H)-one;

9-amino-3,7-phenanthrolin-8(7H)-one;

9-amino-5,6-dihydro-3,7-phenanthrolin-8(7H)-one;

9-(N-acetylamino)-5,6-dihydro-3,7-phenanthrolin-8(7H)-one;

2-amino-4,5,6,7-tetrahydro-3H-benzo[3,4]-cyclohepta [1,2-b]pyridin-3-one;

5,6,7,8-tetrahydro-10-amino-9H-cyclohepta-[1,2-b:4,3-c']dipyridin-9-one;

3-amino-[1]benzothieno[2,3-b]pyridin-2(1H)-one;

3-amino-5-methylfuro[2,3-b:5,4-c']dipyridin-2(1H)-one;

2-amino-9-chloro-4,6-dihydro-3H-thiopyrano-[2,3-b:5,4-b']dipyridin-3-one;

9-(N-acetylamino)-5,7-dihydro-8H-pyrano-[2,3-b:4,5-c']dipyridin-8-one;

9-(N-acetylamino)-6H-thiopyrano[3,2-b:5,4-b']-dipyridin-8(7H)-one;

9-amino-6H-pyrano[2,3-c:5,4-b']dipyridin-8(7H)-one;

10-amino-5,8-dihydrothiepino[2,3-b:4,5-c']-dipyridin-9(6H)-one;

10-amino-5,7-dihydroxepino[3,4-b:5,6-c']-dipyridin-9(8H)-one;

10-amino-6,7-dihydro-1,4-dimethylthiepino[3,2-c:5,4-b']dipyridin-9(8H)-one;

10-amino-5,6-dihydro-4-methoxyoxepino[3,2-b:5,4-b']dipyridin-9(8H)-one;

2-amino-4,6-dihydro-3H[2]-benzopyrano [3,4-b] pyridin-3-one; and

2-(N-acetylamino)-4,6-dihydro-3H[2]benzopyrano[3,4-b]pyridin-3-one

EXAMPLE 12 5,6-Dihydro-benzo[f]-quinolin-3(4H)-one

To a mixture of 60 ml of concentrated sulfuric acid and 15 ml of water, add 22.2 gm of 2-cyano-5,6-dihydro-benzo[f]quinolin-3(4H)-one. Heat the system at reflux for 24 hours. Cool the system and pour the solution into 1 liter of ice/water. Neutralize the solution with 10N NaOH solution. Extract the solution with 300 ml of chloroform and dry the organic solution over anhydrous magnesium sulfate. Filter the solution and remove the solvent by stripping to give the title compound. (m.p. 249-51° C).

Starting from 22.2 gm of 4,6-dihydro-3H[2]benzopyrano [3,4-b]pyridin-3-one-2-carboxylic acid, repeat the procedure to obtain 4,6-dihydro-3H[2]-benzopyrano-[3,4-b]-pyridin-3-one.

EXAMPLE 13 2-Chloro-5,6-dihydro-benzo[f]-quinolin-3(4H)-one

Add 19.7 gm of 5,6-dihydro-benzo[f]quinolin-3(4H)-one to 300 ml of acetic acid. Bubble chlorine into the reaction solution for 4 hours at 100°. Cool the system and collect the product as a precipate. Dissolve the solid into water and neutralize the solution with 1N NaOH. Extract the product with chloroform. Dry the chloroform over anhydrous magnesium sulfate and filter the solution. Remove the solvent by stripping to give the title compound.

Using 4,6-dihydro-3H[2] -benzopyrano-[3,4-b]-pyridin-3-one as starting material, repeat the procedure to produce 2-chloro-4,6-dihydro-3H[2]-benzpyrano[3,4-b]pyridin-3-one.

EXAMPLE 14 2-(N-Methylamino)-5,6-dihydro-benzo[f]quinolin-3(4H)-one

Add 23.1 gm of 2-chloro-5,6-dihydro-benzo[f]-quinolin-3(4H)-one to 100 ml of 40% aqueous methylamine and 250 ml of water. Heat the system in a steel bomb at 140° C for 5 days. Cool to stop the reaction, remove solvent, add water and isolate 2-(N-methylamino)-5,6-dihydro-benzo[f]quinolin-3(4H)-one.

EXAMPLE 15 2-Hydroxy-5,6-dihydro-benzo[f]-quinolin-3(4H)-one

Add 23.1 gm of 2-chloro-5,6-dihydro-benzo[f]-quinolin-3(4H)-one to 300 ml of methanol containing 30 gm of sodium methoxide. Heat the system in a steel bomb at about 200° C for 12 hours. Cool to stop the reaction, remove solvent, treat with water and isolate the title compound.

Similarly, the following compounds of this invention may be prepared by using the above procedures and employing the appropriate starting materials:

10-chloro-5,6,7,8-tetrahydro-9H-cyclohepta-[1,2-b:4,3-c']dipyridin-9-one;

10-bromo-5,6,7,8-tetrahydro-9H-cyclohepta[1,2-b:3,4-b']dipyridin-9-one;

10-chloro-5,6,7,8-tetrahydro-9H-cyclohepta-[1,2-b:3,4-c']dipyridin-9-one;

2-hydroxybenzo[f]quinolin-3(4H)-one;

5,6-dihydro-2-hydroxybenzo[f]quinolin-3(4H)-one;

5,6,7,8-tetrahydro-10-(N,N-dimethylamino)-9H-cyclohepta[1,2-b:4,3-b']dipyridin-9-one;

5,6-dihydro-9-N-methylamino-3,7-phenathrolin-8(7H)-one;

5,6-dihydro-9-(N-ethylamino)-3,7-phenathrolin-8(7H)-one;

5,6,7,8-tetrahydro-10-(N,N-di-n-propyl-amino)-9H-cyclohepta[1,2-b:3,4-c']dipyridin-9-one;

2-(N-methylamino)-4,6-dihydro-3H[2]-benzopyrano[3,4-b] pyridin-3-one; and

2-hydroxy-4,6-dihydro-3H[2]benzopyrano[3,4-b]pyridin-3-one.

EXAMPLE 16 2-cyano-4,6-dihydro-3H[2]-benzothiopyrano[3,4-b]pyridin-3-one-5,5-dioxide

Add 22.6 gm of 2-cyano-4,6-dihydro-3H[2]benzothiopyrano[3,4-b]pyridin-3-one to 150 ml of chloroform. Add 30.0 gm of m-chloroperbenzoic acid to the system. Stir the system at room temperature for 18 hours. Wash the organic solution first with saturated sodium bisulfite solution (2 x 500 ml) and then with saturated sodium bicarbonate solution (2 x 500 ml). Dry the organic solution over anhydrous magnesium sulfate, filter, and remove the chloroform by stripping to give the title compound.

The following formulations exemplify some of the dosage forms in which the compounds of this invention may be employed. In each, the active ingredient is designated by the term "Drug" which is meant to indicate the following compound:

2-cyano-5,6-dihydrobenzo[f]quinolin-3(4H)-one, or

2-cyano-4,6-dihydro-3H-[2]benzopyrano[3,4-b]-pyridine-3-one.

It is contemplated, however, that these compoundsmay be replaced by equally effective quantities of other compounds within the scope of formula I, especially those which are named above. All temperatures are in degrees Celsius.

EXAMPLE 17 Parenteral Dosage Forms

Injectable solution of drug:

| Ingredient | mg/ml |
|---|---|
| Drug | 20 |
| Methylparaben | 0.2 |
| Propylparaben | 1.6 |
| Sodium Bisulfite | 3.2 |
| Disodium Edetate | 0.1 |
| Water for Injection q.s. ad | 1.0 ml |

Method of Manufacture

1. Dissolve parabens in a portion ( 85% of the final volume) of the water for injection at 65-70°.
2. Cool to 25-35°. Charge and dissolve the sodium bisulfite and disodium edetate.
3. Charge and dissolve Drug.
4. Bring the solution to final volume by adding water for injection.
5. Filter the solution through 0.22 membrane and fill into appropriate containers.
6. Terminally sterilize the units by autoclaving.

## CLAIMS

(applicable to all countries other than Austria)

1. A compound of the formula

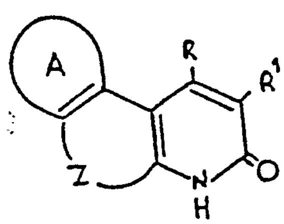

wherein R is hydrogen or lower alkyl;

$R^1$ is hydrogen, hydroxy, lower alkoxy, cyano, lower alkyl, halogen, $-NR^2R^3$, $-CONR^2R^3$, (wherein $R^2$ and $R^3$ are independently hydrogen, lower alkyl, or hydroxy lower alkyl), $-NHCOR^2$, $-COOR^2$ (wherein $R^2$ is as defined above), or

M
‖
$-CNH_2$ wherein M is sulfur or NH; with the proviso that R and $R^1$ are not both hydrogen;

Z is selected from -CH=CH-,

$(CH_2)_n$,

wherein n is 1,2,3 or 4,
$-(CH_2)_p-S(O)_m-(CH_2)_r$, or
$-(CH_2)_p-O-(CH_2)_r$

wherein m is 0,1 or 2, and p and r are

independently 0,1,2 or 3 subject to the proviso that r + p is 0,1,2 or 3; A is a substituted or unsubstituted benzo or pyrido ring which when substituted have 1 to 3 substitutents independently selected from halogen, hydroxy, lower alkyl and lower alkoxy;

and pharmaceutically acceptable salts thereof.

2. A compound as defined in claim 1 characterized in that $R^1$ is hydroxy, lower alkoxy, cyano, $-NR^2R^3$, $-CONR^2R^3$ or $-NHCOR^2$ wherein $R^2$ and $R^3$ are as defined in claim 1.

3. A compound as defined in claim 2, characterized in that R1 is cyano, amino or $-\overset{O}{\overset{\|}{C}}NH_2$.

4. A compound as defined in any one of the preceding claims, characterized in that R is hydrogen or methyl.

5. A compound as defined in any one of the preceding claims, characterized in that R is hydrogen and $R_1$ is cyano.

6. A compound as defined in any one of the preceding claims, characterized in that Z is

$-CH_2CH_2-$, $-CH{=}CH-$ or $-OCH_2-$.

7. A compound as defined in any one of the preceding claims, characterized in that A is a benzo or a 3-pyrido ring.

8. 2-cyano-5,6-dihydro-benzo[f] quinolin-3[4H]-one; 2-cyano-benzo[f] quinolin-3[4H]-one; 9-cyano-5,6-dihydro-3,7-phenanthrolin-8[7H]-one; 9-cyano -3,7-phenanthrolin-8[7H]-one; or 2-cyano-4,6-dihydro-3H-[2]benzopyrano-[3,4-b] pyridin-3-one.

9. A pharmaceutical composition comprising a compound as defined in any one of the preceding claims in admixture with a pharmaceutically acceptable carrier or excipient.

10. A process for making a compound of formula I as defined in claim 1, characterized by reacting a compound of formula II

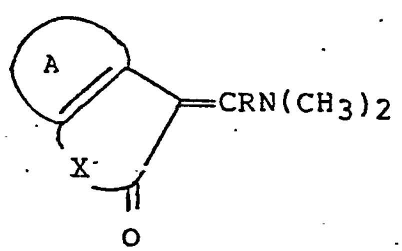

II

in which R and A are as defined in claim 1, and X is $-(CH_2)_n-$, or $-(CH_2)_p-O-(CH_2)_r$ or $-(CH_2)_pS-(CH_2)_r$ wherein n,p and r are as defined in claim 1, with a compound of formula III

$$R^{8}-CH_2-\overset{O}{\overset{\|}{C}}-NH_2 \qquad III$$

in which $R^8$ is hydrogen, cyano, lower alkoxy or lower alkyl under basic conditions to form a compound of formula $I_a$

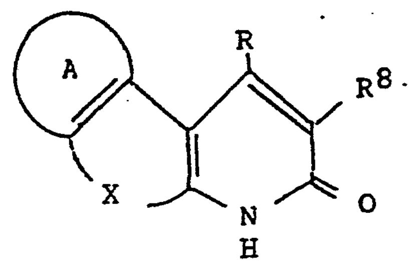

I a

(and when R and $R^8$ are both hydrogen, halogenating the so obtained compound of formula Ia to produce a compound of formula I in which $R^1$ is halogen), whereby a compound of formula I is obtained in which A,R, and X are as defined above and $R^1$ is halogen, hydrogen, cyano, loweralkoxy or loweralkyl, and thereafter if desired, converting such compound of formula I into another compound of formula I by a known method, and isolating the compound of formula I as such or as a pharmaceutically acceptable salt.

## CLAIMS

(applicable for AUSTRIA)

1. A process for preparing a compound of the formula I

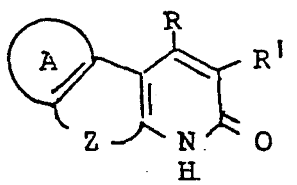

I

wherein R is hydrogen or lower alkyl;

$R^1$ is hydrogen, hydroxy, lower alkoxy, cyano, lower alkyl, halogen, $-NR^2R^3$, $-CONR^2R^3$, (wherein $R^2$ and $R^3$ are independently hydrogen, lower alkyl, or hydroxy-lower-alkyl), $-NRCOR^2$, $-COOR^2$ (wherein $R^2$ is as defined above), or $-\overset{\overset{M}{\|}}{C}NH_2$ wherein M is sulfur or NH; with the proviso that R and $R^1$ are not both hydrogen;

Z is selected from -CH=CH-,

$(CH_2)_n$,

wherein n is 1,2,3 or 4,

$-(CH_2)_p-S(O)_m-(CH_2)_r$. or

Austria

$-(CH_2)_p-O-(CH_2)_r$

wherein m is 0,1 or 2, and p and r are independently 0,1,2 or 3 subject to the proviso that r + p is 0,1,2 or 3; and A is a substituted or unsubstituted benzo or pyrido ring which when substituted have 1 to 3 substitutents independently selected from halogen, hydroxy, lower alkyl and lower alkoxy;

and pharmaceutically acceptable salts thereof, characterized by reacting a compound of formula II

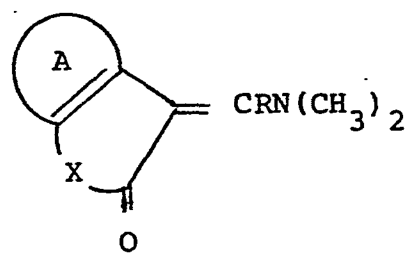

II

in which R and A are as defined in claim 1, and X is $-(CH_2)_n-$, or $-(CH_2)_p-O-(CH_2)_r-$ or $-(CH_2)_p-S-(CH_2)_r-$
wherein n, p and r are as defined in claim 1, with a compound of formula III

$$R^8-CH_2-\overset{O}{\overset{\|}{C}}-NH_2 \qquad III$$

in which $R^8$ is hydrogen, cyano, lower alkoxy or lower alkyl under basic conditions to form a compound of formula $I_a$

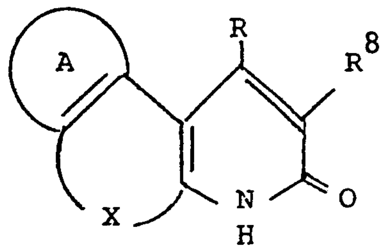

I a

(and when R and $R^8$ are both hydrogen, halogenating the so obtained compound of formula Ia to produce a compound of formula I in which $R^1$ is halogen), whereby a compound of formula I is obtained in which A, R and X are as defined above and $R^1$ is halogen, hydrogen, cyano, loweralkoxy or loweralkyl, and thereafter if desired, converting such compound of formula I into another compound of formula I by a known method, and isolating the compound of formula I as such or as a pharmaceutically acceptable salt.

2. A process as defined in claim 1, characterized in that a compound is produced in which $R^1$ is hydroxy, loweralkoxy, cyano, $-NR^2R^3$, $-CONR^2R^3$, or $-NHCOR^2$ wherein $R^2$ and $R^3$ are as defined above.

3. A process as defined in claim 1 or 2, characterized in that a compound is produced in which $R^1$ is cyano amino or $-\overset{O}{\overset{\|}{C}}NH_2$.

4. A process as defined in claim 1, 2 or 3, characterized in that a compound is produced in which

R is hydrogen or methyl.

5. A process as defined in any one of the preceding claims, characterized in that a compound is produced in which R is hydrogen and $R^1$ is cyano.

6. A process as defined in any one of the preceding claims, characterized in that a compound is produced in which Z is

$-CH_2CH_2-$ , $-CH=CH-$ or $-OCH_2-$ .

7. A process as defined in any one of the preceding claims, characterized in that A is benzo or a 3-pyrido group.

8. A process according to claim 7 characterized in that

2-cyano-5,6-dihydro-benzo[f] quinolin-3[4H]-one;
2-cyano-benzo[f] quinolin-3[4H]-cne;
9-cyano-5,6-dihydrc-3,7-phenanthrolin-8[7H]-one;
9-cyano-3,7-phenanthrolin-8[7H]-one; cr
2-cyano-4,6-dihydro-3H-[2]benzopyrano-[3,4-b] pyridin-3-one, is produced.

9. A process for making a pharmaceutical composition, characterized in that a compound of formula I as defined in claim 1 is admixed with a pharmaceutically acceptable carrier or excipient.

Austria

10. A process for making a pharmaceutical composition, characterized in that a compound of formula I as defined in claim 1, whenever prepared by a process as defined in any one of claims 1 to 8 is admixed with a pharmaceutically acceptable carrier or excipient.

0102046

European Patent Office

# EUROPEAN SEARCH REPORT

Application number: EP 83108240.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US - A - 2 495 048 (AMON)<br>* Claims *<br>-- | 1,6,7 | C 07 D 221/10<br>C 07 D 221/16<br>C 07 D 491/04<br>C 07 D 495/04<br>C 07 D 471/04<br>C 07 D 491/12<br>C 07 D 495/12<br>A 61 K 31/435 |
| A | US - A - 4 180 666 (CHORVAT)<br>* Column 1, lines 1-39 *<br>-- | 1,7,9 | |
| A | EP - A1 - 0 027 904 (SANDOZ)<br>* Abstract *<br>-- | 1,6,7,9 | |
| A | CHEMICAL ABSTRACTS, vol. 92, no. 13, March 31, 1980, Columbus, Ohio USA<br>A. KUMAR et al. "Ketene dithioacetals", page 639, column 1, Abstract-no. 110 776q<br>& J. Chem. Res., Synop., vol. 8, 1979, pages 268-269<br>---- | 1 | |

TECHNICAL FIELDS SEARCHED (Int. Cl. 3)

C 07 D 221/00
C 07 D 471/00
C 07 D 491/00
C 07 D 495/00
A 61 K 31/00

The present search report has been drawn up for all claims

| Place of search | Date of completion of the search | Examiner |
|---|---|---|
| VIENNA | 14-11-1983 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document